# EUROPÄISCHE PATENTSCHRIFT

(11) **EP 0 800 421 B1**
(45) Veröffentlichungstag und Bekanntmachung des Hinweises auf die Patenterteilung: **10.03.1999**
(21) Anmeldenummer: 95942679.2
(22) Anmeldetag: 16.12.1995
(51) Int. Cl.: B01J 25/00, B01J 37/00

(54) **VERFAHREN ZUR HERSTELLUNG EINES HYDRIERKATALYSATORS**
METHOD OF PRODUCING A HYDROGENATION CATALYSER
PROCEDE DE FABRICATION D'UN CATALYSEUR D'HYDROGENATION

(30) Priorität: 27.12.1994 DE 4446907
(43) Veröffentlichungstag der Anmeldung: 15.10.1997
(73) Patentinhaber: BASF AKTIENGESELLSCHAFT, 67056 Ludwigshafen (DE)
(72) Erfinder: VICARI, Maximilian, D-67117 Limburgerhof (DE); FLICK, Klemens, D-76863 Herxheim (DE); MELDER, Johann-Peter, D-67141 Neuhofen (DE); SCHNURR, Werner, D-67273 Herxheim (DE); WULFF-DÖRING, Joachim, D-67227 Frankenthal (DE)
(86) Internationale Anmeldenummer: EP9504986
(87) Internationale Veröffentlichungsnummer: WO9620043

(56) Entgegenhaltungen:
- EP-A- 0 648 534
- US-A- 1 896 320
- US-A- 4 826 799
- CHEMICAL ABSTRACTS, vol. 78, no. 8, 26.Februar 1973 Columbus, Ohio, US; abstract no. 48540p, "JP 47031833" Seite 335; Spalte RH; XP002001563 & JP,A,47 031 833 (TORAY INDUSTRIES, INC) 16.August 1972
- PATENT ABSTRACTS OF JAPAN vol. 13, no. 589 (C-670), 25.Dezember 1989 & JP,A,01 247538 (TOSHIBA CORP.), 3.Oktober 1989,

## Beschreibung

Die vorliegende Erfindung betrifft ein verfahren zur Herstellung eines Hydrierkatalysators auf der Basis einer Legierung aus Aluminium und eines Übergangsmetalles, indem man aus der Legierung und einem Hilfsmittel eine Knetmasse herstellt, die Knetmasse zu Formkörpern formt, die Formkörper calciniert, und die calcinierten Formkörper mit einem Alkalimetallhydroxid behandelt.

Des weiteren betrifft die Erfindung verfahren zur Hydrierung und Hydrogenolyse, insbesondere die partielle Hydrierung von aliphatischen alpha,omega-Dinitrilen zu aliphatischen alpha,omega-Ami-nonitrilen sowie die verwendung der erfindungsgemäß hergestellten Katalysatoren zur Hydrierung und Hydrogenolyse.

In der Ind. Eng. Chem. Res. 1989, 28, S. 1764 bis 1767, wird ein Verfahren zur Herstellung von Katalysatoren des Raney-Typs beschrieben. Nach diesem verfahren wird eine Legierung aus Aluminium und Nickel mit polyethylen und Mineralöl bei 150°C verknetet, anschließend zu Formkörpern extrudiert und danach wird das Mineralöl mit Hexan extrahiert. Die so erhaltenen Formkörper werden sodann bei 900 bis 1200°C in Luft calciniert, wobei ein Teil des Aluminiums zu Aluminiumoxid oxidiert wird. Aus den calcinierten Formkörpern erhält man Katalysatoren, indem man die calcinierten Formkörper mit einem Alkalimetallhydroxid behandelt, wobei der größte Teil des nicht oxidierten Aluminiums aus den Formkörpern herausgelöst wird. Nachteilig an dieser Verfahrensweise ist die Verwendung relativ großer Mengen an Mineralöl (20 Gew.-%), das zudem in einem verfahrensschritt vor der Calcinierung mit einer weiteren Substanz, dem Extraktionsmittel Hexan, entfernt werden muß. Des weiteren ist die Temperatur beim Kneten mit 150°C für kommerzielle Anwendungen u.a. wegen des Energieverbrauchs zu hoch.

Aufgabe der vorliegenden Erfindung war es daher, ein Verfahren bereitzustellen, das die genannten Nachteile nicht aufweist. Insbesondere sollte eine Verfahrensvereinfachung gegenüber dem Stand der Technik erreicht werden, indem man keinen Zwischenschritt zur Entfernung des Hilfsmittels benötigt.

Demgemäß wurde ein verbessertes verfahren zur Herstellung eines Hydrierkatalysators auf der Basis einer Legierung aus Aluminium und eines Übergangsmetalles gefunden, indem man aus der Legierung und einem Hilfsmittel eine Knetmasse herstellt, die Knetmasse zu Formkörpern formt, die Formkörper calciniert, und die calcinierten Formkörper mit einem Alkalimetallhydroxid behandelt, wobei man als Hilfsmittel (a) Polyvinylalkohol und Wasser oder (b) Stearinsäure einsetzt.

Des weiteren wurden verfahren zur Hydrierung und Hydrogenolyse, insbesondere die partielle Hydrierung von aliphatischen alpha,omega-Dinitrilen zu aliphatischen alpha,omega-Aminonitrilen sowie die Verwendung der erfindungsgemäß hergestellten Katalysatoren zur Hydrierung und Hydrogenolyse gefunden.

Nach dem erfindungsgemäßen verfahren stellt man zunächst aus einer Legierung aus Aluminium und eines Übergangsmetalles und einem Hilfsmittel eine Knetmasse her.

Als Übergangsmetalle kann man bevorzugt Nickel, Cobalt, Eisen, Kupfer, besonders bevorzugt Nickel und Cobalt, einsetzen.

Die Herstellung der Aluminium-Legierung erfolgt in an sich bekannter Weise, beispielsweise nach dem in der DE-A 21 59 736 beschrieben Verfahren. Das Massenverhältnis von Aluminium zu Übergangsmetall in der Legierung wählt man in der Regel im Bereich von 35:1 bis 80:1, bevorzugt von 50:1 bis 70:1.

Als Hilfsmittel setzt man erfindungsgemäß entweder (a) Polyvinylalkohol und Wasser oder (b) Stearinsäure ein.

Üblicherweise verwendet man Polyvinylalkohol mit einem Molekulargewicht im Bereich von 3000 bis 6000, vorzugsweise von 4500 bis 5500 g/mol.

Das Gewichtsverhältnis von Polyvinylalkohol zu Wasser wählt man im allgemeinen im Bereich von 0,3:1 bis 0,4:1, vorzugsweise von 0,35:1 bis 0,37:1. Außerhalb dieses Bereiches läßt sich nach bisherigen Beobachtungen die Knetmasse schlechter bis gar nicht zu Formkörpern verarbeiten.

In einer bevorzugten Ausführungsform mischt man zunächst die Legierung mit dem üblicherweise festen polyvinylalkohol und gibt dann portionsweise solange Wasser zu, bis man eine gut formbare, plastische Knetmasse erhält.

Bei Verwendung von Polyvinylalkohol und Wasser als Hilfsmittel, wählt man in der Regel das Gewichtsverhältnis von Legierung zu Polyvinylalkohol im Bereich von 20:1 bis 80:1, wobei man die Herstellung der Knetmasse üblicherweise bei einer Temperatur im Bereich von 10 bis 40, vorzugsweise von 25 bis 35°C vornimmt.

Üblicherweise wählt man das Gewichtsverhältnis von Stearinsäure zu Legierung im Bereich von 0,01:1 bis 1,0:1, vorzugsweise von 0,04:1 bis 0,06:1, wobei man die Herstellung der Knetmasse üblicherweise bei einer Temperatur im Bereich von 70 bis 140, vorzugsweise von 75 bis 85°C vornimmt.

Die Herstellung der Knetmasse kann man in an sich üblicher Art und Weise durchführen, beispielsweise in einer entsprechenden Misch- oder Knetvorrichtung.

Erfindungsgemäß stellt man aus der Knetmasse aus im wesentlichen Legierung und Hilfsmittel Formkörper her. Die räumliche Ausgestaltung der Formkörper sowie ihre Herstellung sind nach bisherigen Beobachtungen für den Erfolg der Erfindung nicht ausschlaggebend. Bevorzugte Formkörper sind beispielsweise Tabletten und Stränge. Die Verarbeitung zu den Formkörper nimmt man üblicherweise in hierfür bekannten Apparaturen vor, beispielsweise in Extrudern, Tabletten- oder Strangpressen.

Bei der Verarbeitung in Extrudern wählt man üblicherweise ein L/ D-Verhältnis im Bereich von 10:1 bis 2:1, vorzugsweise von 3:1 bis 5:1, eine Temperatur im Bereich von 10 bis 40, vorzugsweise von 25 bis 35°C und einen Druck im Bereich von 10 bis 20, vorzugsweise von 12,5 bis 17,5 MPa.

In einer besonderen Ausführungsform stellt man Stränge mit einem Durchmesser von 1,5 mm und einer Länge von 5 mm her, wobei die Herstellung in der Regel so vorgenommen wird, daß die so erhaltenen Stränge sofort nach dem Austreten aus dem Extruder einer Temperatur im Bereich von 100 bis 200°C zum Antrocknen für einen Zeitraum im Bereich von 0,2 bis 2 min ausgesetzt werden. Anschließend erfolgt eine zwölfstündige Trocknung bei 120°C.

Die Calcinierung der Formkörper nimmt man üblicherweise bei Temperaturen im Bereich von 700 bis 1200, vorzugsweise von 750 bis 900°C vor, wobei die verweilzeit in der Regel im Bereich von 0,5 bis 3, vorzugsweise von 0,9 bis 1,1 h beträgt. In einer besonderen Ausführungsform tempert man die Formkörper zunächst eine Stunde bei 750°C und erhöht dann die Temperatur für zwei Stunden auf 900°C.

Die Calcinierung nimmt man üblicherweise bei Atmosphärendruck in Luft vor.

Die Aktivierung der calcinierten Formkörper nimmt man erfindungsgemäß mit einem Alkalimetallhydroxid wie Lithiumhydroxid, Natriumnydroxid, Kaliumhydroxid, Cäsiumhydroxid, bevorzugt Natriumhydroxid, oder Mischungen davon. In der Regel setzt man eine wäßrige Lösung des Alkalimetallhydroxids, insbesondere Natronlauge, ein, wobei das Gewichtsverhältnis von Wasser zu Alkalimetallhydroxid im allgemeinen im Bereich von 10:1 bis 30:1, vorzugsweise von 15:1 bis 25:1 liegt. Das Mol-Verhältnis von Alkalimetallhydroxid zu Aluminium wählt man in der Regel im Bereich von 1:1 bis 4:1, vorzugsweise von 1,5:1 bis 2,5:1. Die Temperatur der Aktivierung wählt man üblicherweise im Bereich von 25 bis 95, vorzugsweise von 45 bis 80°C. Die Dauer der Aktivierung hängt im wesentlichen vom gewünschten Endgehalt an Aluminium ab und liegt üblicherweise im Bereich von 10 bis 30, bevorzugt von 15 bis 25 h. Zweckmäßig kontrolliert man die Aktivierung durch Messung der bei der Aktivierung freigesetzten Wasserstoffmenge.

Nach der Aktivierung wäscht man üblicherweise die aktivierten und calcinierten Formkörper mit Wasser, bevorzugt bis ein pH-Wert des Waschwassers von mindestens 8,0 erreicht ist, und bewahrt Formkörper unter Wasser, bevorzugt in einer Mischung aus Wasser und Methanol, auf.

Die erfindungsgemäß hergestellten Katalysatoren kann man zur Hydrierung und zur Hydrogenolyse einsetzen wie zur Hydrierung von C-C- und C-N-Doppel- und Dreifachbindungen, von Ketonen, Alkoholen, zur Etherspaltung, zur Reduktion von Nitroverbindungen und Oximen, zur Herstellung von sekundären Aminen aus Ketonen und primären Aminen, zur Dehalogenierung und zur Reduktion von Thioketonen.

In einer bevorzugten Ausführungsform setzt man die erfindungsgemäß hergestellten Katalysatoren zur partiellen Hydrierung von aliphatischen alpha,omega-Dinitrilen zu aliphatischen alpha, omega-Aminonitrilen.

Die partielle Hydrierung kann sowohl in Gas - als auch in Flüssigphase in einem Rohrreaktor sowohl in Sumpf - als auch in Rieselfahrweise, bevorzugt in Rieselfahrweise, durchgeführt werden.

Als Ausgangsstoffe kann man aliphatische alpha,omega-Dinitrile der allgemeinen Formel I

NC-(CH₂)ₙ-CN I

in der n eine ganze Zahl von 1 bis 10, insbesondere 2, 3, 4, 5 und 6, bedeutet, eingesetzen. Besonders bevorzugte Verbindungen I sind Bernsteinsäuredinitril, Glutarsäuredinitril, Adipinsäuredinitril ("Adiponitril"), Pimelinsäuredinitril und Korksäuredinitril ("Süberonitril"), ganz besonders bevorzugt Adiponitril. Nach dem bevorzugten verfahren werden die vorstehend beschriebenen Dinitrile I in Gegenwart eines Lösungsmittels unter Verwendung des erfindungsgemäß hergestellten Katalysators partiell zu alpha,omega-Aminonitrilen der allgemeinen Formel II

NC-(CH₂)ₙ-CH₂-NH₂ II

hydriert, wobei n die vorstehend genannte Bedeutung hat. Besonders bevorzugte Aminonitrile II sind solche, in denen n einen Wert von 2, 3, 4, 5 oder 6 hat, insbesondere 4, d.h. 4-Aminobutansäurenitril, 5-Aminopentansäurenitril, 6-Aminohexansäurenitril ("6-Aminocapronitril") , 7-Aminoheptansäurenitril und 8-Aminooctansäurenitril, ganz besonders bevorzugt 6-Aminocapronitril.

Die partielle Hydrierung in der Flüssigphase führt man bevorzugt bei einer Temperatur im Bereich von 20 bis 150, vorzugsweise von 30 bis 90°C und einen Druck in der Regel im Bereich von 2 bis 30, vorzugsweise von 3 bis 20 MPa durch. Besonders bevorzugt führt man die partielle Hydrierung in Gegenwart eines Lösungsmittels, bevorzugt Ammoniak, Amine, Diamine und Triamine mit 1 bis 6 C-Atomen wie Trimethylamin, Triethylamin, Tripropylamin oder Tributylamin, oder Alkohole wie Methanol und Ethanol, besonders bevorzugt Ammoniak durch. In einer bevorzugten Ausführungsform wählt man einen Gehalt an Ammoniak im Bereich von 1 bis 10, bevorzugt von 2 bis 6 g pro g Adipodinitril. Bevorzugt wählt man dabei eine Katalysatorbelastung im Bereich von 0,1 bis 2,0, bevorzugt von 0,3 bis 1,0 kg Adipodinitril/l*h. Auch hier kann man durch Veränderung der Verweilzeit den Umsatz und damit die Selektivität gezielt einstellen.

Bei der partiellen Hydrierung in der Gasphase hält man im allgemeinen eine Katalysatorbelastung von 0,03 bis 10, vorzugsweise 0,05 bis 3 kg Dinitril je kg Katalysator und Stunde ein.

Die Wasserstoffkonzentration im Eingangsgas richtet sich üblicherweise nach der Dinitrilkonzentration. Das Molverhältnis von Wasserstoff zu Dinitril beträgt in der Regel 2:1 bis 300:1, bevorzugt 10:1 bis 200:1.

Die Umsetzung kann in der Gasphase, mit oder ohne Lösungsmittel, kontinuierlich als Festbettreaktion mit fest angeordnetem Katalysator, beispielsweise in Sumpf- oder Rieselfahrweise oder als Wirbelbettreaktion mit in auf- und abwirbelnder Bewegung befindlichem Katalysator durchgeführt werden. Bevorzugt ist das Arbeiten im Festbett. Durch Veränderung der verweilzeit kann der Umsatz und damit die Selektivität gezielt eingestellt werden.

Bei der partiellen Hydrierung in der Gasphase arbeitet man üblicherweise bei Temperaturen im Bereich von 100 bis 250, vorzugsweise von 150 bis 220, insbesondere von 160 bis 200°C und bei Drücken im Bereich von 0,1 bis 30, bevorzugt 0,7 bis 10, besonders bevorzugt 0,9 bis 5 bar.

Nach dem bevorzugten Verfahren erhält man alpha,omega-Aminonitrile in guten Selektivitäten und mit nur geringen Mengen an Hexamethylendiamin. Des weiteren weisen die erfindungsgemäß eingesetzten Katalysatoren eine deutlich längere Standzeit auf als vergleichbare Katalysatoren aus dem Stand der Technik. Die alpha,omega-Aminonitrile sind wichtige Ausgangsverbindungen zur Herstellung von cyclischen Lactamen, insbesondere 6-Aminocapronitril für Caprolactam.

Die Vorteile des erfindungsgemäßen Verfahrens liegen in einer einfacheren und weniger Energie verbrauchenden Verfahrensweise verglichen mit verfahren des Standes der Technik.

### Beispiele

### Beispiel 1

### Herstellung eines Aluminium-Nickel-Katalysators

Zu einer Mischung aus 800 g eines Pulvers, bestehend aus einer Legierung aus 48 Gew.-% Nickel und 52 Gew.-% Aluminium (hergestellt analog zu Beispiel 1 aus der DE-A 21 59 736) und 33 g eines Polyvinylalkohols (Molekulargewicht = 5000 g/mol), wurden portionsweise 90 ml Wasser und anschließend drei Stunden in einem Kneter geknetet. Danach wurde die so erhaltene Knetmasse bei einem Druck von 15 MPa in einem Extruder bei Raumtemperatur zu 1,5 mm dicken und 5 mm langen Strängen verarbeitet. Die erhaltenen Stränge wurden bei einer Temperatur von 120°C 2 min angetrocknet und dann 12 h bei 120°C gehalten. Die Calcinierung erfolgte zunächst bei 750°C (eine Stunde) und anschließend bei 900°C (zwei Stunden).

Zur Aktivierung wurden 500 g der so hergestellten Stränge mit 1,5 l einer 20 gew.-%igen NaOH-Lösung bei 90°C versetzt. Nach 26 h (die entstandene Wasserstoffmenge betrug 143,2 l) wurden die Stränge-mit Wasser gewaschen, wobei der pH-Wert des letzten Waschwassers 7,5 betrug.

### Beispiel 2

### Herstellung von Tabletten mit Stearinsäure als Hilfsmittel

2250 g eines Al-Ni-Legierungs-Pulvers (es wurde das gleiche Pulver wie in Beispiel 1 verwendet) wurde auf 80°C erwärmt und mit 112,5 g flüssiger Stearinsäure vermischt. Nach dem Abkühlen wurde die erhaltene feste Masse durch ein Sieb mit einer Maschenweite von 1 mm gedrückt unter Erhalt eines Pulvers. Dieses Pulver wurde bei Raumtemperatur auf einer Tablettiermaschine tablettiert (3 mm Durchmesser, 3 mm Höhe).

Die so erhaltenen Tabletten wurden bei 900°C zwei Stunden calciniert. Zur Aktivierung wurden 411 g der Tabletten mit 1,5 l einer 20 gew.-%igen Natronlauge bei 90°C versetzt. Nach 24 h wurde abgekühlt und 30 h mit Wasser gewaschen (der pH-Wert des letzten Waschwassers betrug 8,0).

### Beispiel 3

### Partielle Hydrierung

Durch einen Reaktor von 55 cm Länge und 1,5 cm Innendurchmesser, befüllt mit 80 ml (156 g) des nach Beispiel 1 erhaltenen Katalysators, wurde bei 18 MPa und 35°C eine Mischung aus 55 ml/h Adiponitril, 120 ml/h flüssig Ammoniak und 200 l/h Wasserstoff geleitet.

Bei einem 56%igen Umsatz setzte sich die Reaktionsmischung aus 44 Gew.-% Adiponitril, 44 Gew.-% 6-Aminocapronitril und 12 Gew.-% Hexamethylendiamin zusammen. Durch Erhöhung der Temperatur auf 40°C stieg der Umsatz auf 68%. Die Reaktionsmischung setzte sich aus 32 Gew.-% Adiponitril, 51 Gew.-% 6-Aminocapronitril und 17 Gew.-% Hexamethylendiamin zusammen.

### Beispiel 4

### Gasphasenhydrierung

Adipodinitril und Wasserstoff wurden bei einer Reaktionstemperatur von 180°C und einem Wasserstoff/Adipodinitril-Molverhältnis von 50:1 über 100 ml des Katalysators aus Beispiel 1 in Rieselfahrweise geleitet, wobei die Katalysatorbelastung 0,15 g ADN/g Katalysator und Stunde betrug. Der gasförmige Reaktionsaustrag wurde in Kühlfallen kondensiert und gaschromatographisch analysiert. Die 6-Aminocapronitril-Ausbeute betrug 53 % (Selektivität 72 %, Umsatz 74 %), die Ausbeute an HMD 6 %.

## Patentansprüche

1. Verfahren zur Herstellung eines Hydrierkatalysators auf der Basis einer Legierung aus Aluminium und eines Übergangsmetalles, indem man aus der Legierung und einem Hilfsmittel eine Knetmasse herstellt, die Knetmasse zu Formkörpern formt, die Formkörper calciniert, und die calcinierten Formkörper mit einem Alkalimetallhydroxid behandelt, dadurch gekennzeichnet, daß man als Hilfsmittel (a) Polyvinylalkohol und Wasser oder (b) Stearinsäure einsetzt.

2. Verfahren nach Anspruch 1, dadurch gekennzeichnet, daß man als Übergangsmetall Nickel, Cobalt, Eisen oder Kupfer einsetzt.

3. Verfahren nach Anspruch 1 oder 2, dadurch gekennzeichnet, daß man die Knetmasse bei einer Temperatur im Bereich von 70 bis 140°C herstellt.

4. Verfahren nach den Ansprüchen 1 bis 3, dadurch gekennzeichnet, daß man das Hilfsmittel nicht vor dem Calcinieren entfernt.

5. Verfahren nach den Ansprüchen 1 bis 4, dadurch gekennzeichnet, daß man das Gewichtsverhältnis von Polyvinylalkohol zu Wasser im Bereich von 0,3:1 bis 0,4:1 wählt.

6. Verfahren nach den Ansprüchen 1 bis 5, dadurch gekennzeichnet, daß man das Gewichtsverhältnis von Legierung zu Polyvinylalkohol im Bereich von 20:1 bis 80:1 wählt.

7. Verfahren nach den Ansprüchen 1 bis 6, dadurch gekennzeichnet, daß das Polyvinylalkohol ein Molekulargewicht im Bereich von 3000 bis 6000 g/mol aufweist.

8. Verfahren zur Hydrierung und Hydrogenolyse in an sich bekannter Weise, dadurch gekennzeichnet, daß man einen Katalysator, hergestellt gemäß den Ansprüchen 1 bis 7, verwendet.

9. Verfahren zur Herstellung von aliphatischen alpha,omega-Aminonitrilen durch partielle Hydrierung von aliphatischen alpha,omega-Dinitrilen bei erhöhter Temperatur in Gegenwart eines Lösungsmittels und eines Katalysators, dadurch gekennzeichnet, daß man einen Hydrierkatalysator auf der Basis einer Legierung aus Aluminium und eines Übergangsmetalles einsetzt, erhältlich, indem man aus der Legierung und einem Hilfsmittel eine Knetmasse herstellt, dann die Knetmasse zu Formkörpern formt, anschließend die Formkörper calciniert, und schließlich die calcinierten Formkörper mit einem Alkalimetallhydroxid behandelt, mit der Maßgabe, daß man als Hilfsmittel (a) Polyvinylalkohol und Wasser oder (b) Stearinsäure einsetzt.

10. Verwendung eines Katalysators, hergestellt gemäß den Ansprüchen 1 bis 7, zur Hydrierung und Hydrogenolyse.

## Claims

1. A process for the preparation of a hydrogenation catalyst based on an alloy of aluminum and of a transition metal by preparing a kneaded material from the alloy and an assistant, converting the kneaded material into moldings, calcining the moldings and treating the calcined moldings with an alkali metal hydroxide, wherein the assistant used is (a) polyvinyl alcohol and water or (b) stearic acid.

2. A process as claimed in claim 1, wherein the transition metal used is nickel, cobalt, iron or copper.

3. A process as claimed in claim 1 or 2, wherein the kneaded material is prepared at from 70 to 140°C.

4. A process as claimed in any of claims 1 to 3, wherein the assistant is not removed before the calcination.

5. A process as claimed in any of claims 1 to 4, wherein the weight ratio of polyvinyl alcohol to water is chosen to be from 0.3:1 to 0.4:1.

6. A process as claimed in any of claims 1 to 5, wherein the weight ratio of alloy to polyvinyl alcohol is chosen to be from 20:1 to 80:1.

7. A process as claimed in any of claims 1 to 6, wherein the polyvinyl alcohol has a molecular weight of from 3000 to 6000 g/mol.

8. A process for hydrogenation and hydrogenolysis in a manner known per se, wherein a catalyst prepared as claimed in any of claims 1 to 7 is used.

9. A process for the preparation of aliphatic alpha,omega-aminonitriles by partial hydrogenation of aliphatic alpha, omegadinitriles at elevated temperatures in the presence of a solvent and of a catalyst, wherein the hydrogenation catalyst used is based on an alloy of aluminum and of a transition metal and is obtainable by preparing a kneaded material from the alloy and an assistant, then converting the kneaded material into moldings, then calcining the moldings and finally treating the calcined moldings with an alkali metal hydroxide, with the proviso that the assistant used is (a) polyvinyl alcohol and water or (b) stearic acid.

10. Use of a catalyst, prepared as claimed in any of claims 1 to 7, for hydrogenation and hydrogenolysis.

## Revendications

1. Procédé de préparation d'un catalyseur d'hydrogénation à base d'un alliage de l'aluminium et d'un métal de transition, conformément auquel on prépare une masse à malaxer à partir de l'alliage et d'un adjuvant, on moule la masse à malaxer en articles moulés, on calcine les articles moulés et on traite les articles moulés calcinés par un hydroxyde de métal alcalin, caractérisé en ce que l'on utilise, à titre d'adjuvant, (a) du poly(alcool vinylique) et de l'eau ou (b) de l'acide stéarique.

2. Procédé suivant la revendication 1, caractérisé en ce que, à titre de métal de transition, on utilise du nickel, du cobalt, du fer ou du cuivre.

3. Procédé suivant la revendication 1 ou 2, caractérisé en ce que l'on prépare la masse à malaxer à une température qui varie de 70 à 140°C.

4. Procédé suivant les revendications 1 à 3, caractérisé en ce que l'on n'élimine pas l'adjuvant avant la calcination.

5. Procédé suivant les revendications 1 à 4, caractérisé en ce que l'on choisit le rapport pondéral du poly(alcool vinylique) à l'eau dans la plage de 0,3:1 à 0,4:1.

6. Procédé suivant les revendications 1 à 5, caractérisé en ce que l'on choisit le rapport de mélange de l'alliage au poly(alcool vinylique) dans la plage de 20:1 à 80:1.

7. Procédé suivant les revendications 1 à 6, caractérisé en ce que le poly(alcool vinylique) présente un poids moléculaire qui varie de 3000 à 6000 g/mole.

8. Procédé d'hydrogénation et d'hydrogénolyse d'une manière en soi connue, caractérisé en ce que l'on utilise un catalyseur préparé suivant l'une quelconque des revendications 1 à 7.

9. Procédé de préparation d'alpha,oméga-aminonitriles aliphatiques par l'hydrogénation partielle d'alpha,oméga-dinitriles aliphatiques à température élevée et en présence d'un solvant et d'un catalyseur, caractérisé en ce que l'on utilise un catalyseur d'hydrogénation à base d'un alliage de l'aluminium et d'un métal de transition, que l'on peut obtenir pour autant que l'on prépare une masse à malaxer à partir de l'alliage et d'un adjuvant, puis que l'on moule la masse à malaxer en articles moulés, on calcine ensuite les articles moulés et on traite finalement les articles moulés calcinés par un hydroxyde de métal alcalin, avec la condition que l'on utilise, à titre d'adjuvant, (a) du poly(alcool vinylique) et de l'eau ou (b) de l'acide stéarique.

10. Utilisation d'un catalyseur préparé suivant les revendications 1 à 7, en vue de l'hydrogénation et de l'hydrogénolyse.
